# EUROPÄISCHE PATENTANMELDUNG

(11) **EP 4 678 147 A1**
(43) Veröffentlichungstag der Anmeldung: **14.01.2026**
(21) Anmeldenummer: 25187558.9
(22) Anmeldetag: 04.07.2025
(51) Int. Cl.: A61F 5/41

(54) **VORRICHTUNG ZUR VERLÄNGERUNG UND/ODER BEGRADIGUNG EINES PENIS**

(30) Priorität: 10.07.2024 DE 102024119638
(71) Anmelder: MSP Concept GmbH & Co. KG, 10719 Berlin (DE)
(72) Erfinder: Suchy, Matthias, 10719 Berlin (DE)
(74) Vertreter: Adares PartGmbB

(57) **Zusammenfassung**

Vorrichtung zur Verlängerung und/oder Begradigung eines Penis, enthaltend
- ein Befestigungselement (1), das ausgebildet ist, einen Penis zu befestigen,
- eine Zugvorrichtung (2), die mit dem Befestigungselement verbunden ist und ausgebildet ist, eine Zugkraft auf zumindest einen Teil des Penis auszuüben,
- eine Zugkraftmessvorrichtung (3), die ausgebildet ist, die bei Betrieb auf den Penis ausgeübte Zugkraft zu messen,
- ein Elektronikbauteil (5), das ausgebildet ist, eine der gemessenen Zugkraft zugeordnete Klasse aus einer Mehrzahl an Klassen zu ermitteln, wobei die mehreren Klassen eine Klasse umfassen, welche eine ihr zugeordnete Zugkraft als für den Betrieb angemessen klassifiziert,
- eine mit dem Befestigungselement und/oder der Zugvorrichtung verbundene Ausgabeeinrichtung (7), die ausgebildet ist, die von dem Elektronikbauteil ermittelte Klasse haptisch, akustisch und/oder visuell auszugeben, wenn sie die gemessene Zugkraft als für den Betrieb angemessen klassifiziert, und
- eine Stromquelle (6) zum Betreiben des Elektronikbauteils, der Zugkraftmessvorrichtung und der Ausgabeeinrichtung.

## Beschreibung

Die Erfindung betrifft eine Vorrichtung zur Verlängerung und/oder Begradigung eines Penis. Insbesondere betrifft die Erfindung eine Vorrichtung zur dauerhaften Verlängerung und/oder Begradigung eines Penis, mit deren Hilfe es ohne operativen Eingriff möglich ist, auf Basis einer dauernden Dehnung des Penis durch Einwirkung einer Streckkraft eine bleibende Verlängerung und/oder eine bleibende Begradigung zu erzielen. Solche Vorrichtungen werden auch als Penisextensionsgeräte bezeichnet.

Aus der EP 1 480 586 A1 und der DE 10 2020 105 224 A1 sind derartige Vorrichtungen bekannt. Die Vorrichtungen enthalten ein Befestigungselement, das ausgebildet ist, einen Penis zu befestigen, eine Zugvorrichtung in Form von Streckstangen, die mit dem Befestigungselement verbunden ist und ausgebildet ist, eine Zugkraft auf zumindest einen Teil des Penis auszuüben. Das Befestigungselement und die Zugvorrichtung bilden zusammen eine Penisverlängerungs- und/oder -begradigungseinrichtung aus, die die Verlängerung und/oder Begradigung des Penis bewirkt.

Aus der EP 3 463 218 B1 ist ferner eine Befestigungseinrichtung bekannt, die ein Kupplungselement und eine Zugkraftmessvorrichtung enthält und die geeignet ist, eine Zugkraft einer Penisextensionsvorrichtung auf einen Penis zu übertragen, wobei ein Elektronikbauteil und eine Stromquelle vorhanden sind. Die Zugkraftmessvorrichtung ist ausgebildet, einen Messwert für den ausgeübten Zug zusammen mit dem Elektronikbauteil elektrisch oder elektronisch zu ermitteln. Das Elektronikbauteil weist eine Funkvorrichtung zur Übermittlung des ermittelten Messwertes an einen externen Empfänger auf, wobei die Funkvorrichtung als Bluetooth-Vorrichtung ausgebildet ist.

Es besteht aber weiterhin ein Bedarf, eine benutzerfreundliche Penisverlängerungs- und/oder -begradigungseinrichtung bereitzustellen, die eine schmerzfreie und wirksame Benutzung ermöglicht und einfach zu handhaben ist.

Es ist eine Aufgabe der Erfindung, eine Vorrichtung zur Verlängerung und/oder Begradigung eines Penis bereitzustellen, die schmerzfrei und für die körperliche Unversehrtheit unbedenklich zur dauerhaften Verlängerung und/oder Begradigung eines Penis geeignet ist. Gleichzeitig soll ihre Handhabung einfach und bequem sein.

Erfindungsgemäß wird die Aufgabe durch eine Vorrichtung mit den Merkmalen des Patentanspruchs 1 gelöst. Vorteilhafte Weiterbildungen und Modifikationen sind in den Unteransprüchen angegeben.

Die Erfindung betrifft eine Vorrichtung zur Verlängerung und/oder Begradigung eines Penis, enthaltend
- ein Befestigungselement, das ausgebildet ist, einen Penis zu befestigen,
- eine Zugvorrichtung, die mit dem Befestigungselement verbunden ist und ausgebildet ist, eine Zugkraft oder Zugspannung auf zumindest einen Teil des Penis auszuüben,
- eine Zugkraftmessvorrichtung, die ausgebildet ist, die bei Betrieb auf den zumindest einen Teil des Penis ausgeübte Zugkraft zu messen,
- ein Elektronikbauteil, das ausgebildet ist, eine der gemessenen Zugkraft zugeordnete Klasse aus einer Mehrzahl an Klassen zu ermitteln, wobei die mehreren Klassen eine Klasse umfassen, welche eine ihr zugeordnete Zugkraft als für den Betrieb angemessen klassifiziert,
- eine mit dem Befestigungselement und/oder der Zugvorrichtung verbundene Ausgabeeinrichtung, die ausgebildet ist, die von dem Elektronikbauteil ermittelte Klasse haptisch, akustisch und/oder visuell auszugeben, wenn sie die gemessene Zugkraft als für den Betrieb angemessen klassifiziert,
- eine Stromquelle, zum Betreiben des Elektronikbauteils, der Zugkraftmessvorrichtung und der Ausgabeeinrichtung.

Die Ausgabeeinrichtung ist insbesondere mechanisch mit der Zugkraftmessvorrichtung verbunden und somit insbesondere ein Teil der Vorrichtung. Somit wird einem Benutzer der Vorrichtung ermöglicht, zu verifizieren, dass die ausgeübte Zugkraft schmerzfrei und für die körperliche Unversehrtheit unbedenklich zur dauerhaften Verlängerung und/oder Begradigung eines Penis geeignet ist. Dadurch kann der Benutzer sicher sein, dass er auch durch dauerhafte Benutzung keine körperlichen Schäden und/oder Beschwerden haben wird. Es ist üblich, dass der Benutzer solch einer Vorrichtung diese möglichst mehrere Stunden am Tag trägt. Daher ist es für den Benutzer wesentlich, zu wissen, ob die ausgeübte Zugkraft auch für eine längere Zeitdauer geeignet ist. Ferner ist es für den Benutzer auch hilfreich, sicherzustellen, dass die eingestellte Zugkraft ausreichend ist, um die von ihm gewünschte Wirkung zu erzielen.

In einer bevorzugten Ausführungsform ist die Ausgabeeinrichtung weiterhin ausgebildet, die von dem Elektronikbauteil ermittelte Klasse akustisch und/oder visuell auszugeben, wenn sie die gemessene Zugkraft als für den Betrieb unangemessen klassifiziert. Die Ausgabe der ermittelten Klasse, die die gemessene Zugkraft als für den Betrieb unangemessen klassifiziert, unterscheidet sich akustisch und/oder visuell von der Ausgabe der Klasse, die die gemessene Zugkraft als für den Betrieb angemessen klassifiziert. Dadurch wird der Benutzer aktiv darauf hingewiesen, ob die ausgeübte Zugkraft zur dauerhaften Benutzung der Vorrichtung ungeeignet oder geeignet ist. Die akustische und/oder visuelle Unterscheidung kann durch die Ausgabe unterschiedlicher Farben, Symbole, Töne, Tonreihenfolgen und dgl. realisiert sein.

Alternativ oder zusätzlich zu der akustischen und/oder visuellen Ausgabe kann die Ausgabeeinrichtung ausgebildet sein, ein haptisches Signal auszugeben. Haptische Ausgabevorrichtungen umfassen jegliche Vorrichtungen, die taktile Rückmeldungen geben, welche durch den Tastsinn wahrgenommen werden. Dazu gehören Vibrationen, Kräfte, die auf die Haut ausgeübt werden, oder elektrische Impulse, die durch Reizstrom erzeugt werden. Solche Vorrichtungen können in verschiedenen Anwendungen eingesetzt werden, darunter mobile Geräte, Spielkonsolen, medizinische Geräte und Virtual-Reality-Systeme, um ein haptisches Feedback zu liefern.

Bevorzugt ist die Ausgabeeinrichtung als eine optische Anzeigeeinrichtung ausgebildet, die ausgebildet ist, die ermittelte Klasse mittels einer optischen Codierung auszugeben, die Farb- und/oder Symbolinformationen aufweist. Es kann nur der Klasse, die einer als die für den Betrieb angemessen klassifizierte Zugkraft zugeordnet ist, die optische Codierung zugewiesen sein. Bevorzugt ist jeder ermittelbaren Klasse jeweils eine individualisierende optische Codierung zugewiesen. Durch eine derartige optische Codierung sind die Klassen leicht unterscheidbar. Die optische Codierung kann insbesondere in Form von Nummerischen oder Alphanummerischen Zeichen erfolgen, beispielsweise mittels eines Displays.

Wie bereits erwähnt, ist das Elektronikbauteil ausgebildet, für die gemessene Zugkraft eine Klasse zu ermitteln, welche dieser Zugkraft zugeordnet ist. Die Zuordnung bzw. das Ermitteln kann beispielsweise mittels festverdrahteten Bauelementen erfolgen. Wenn beispielsweise die Zugkraft im Elektronikbauteil durch eine gemessene Spannung oder Stromstärke repräsentiert wird, dann kann mittels Schwellwertvergleich eine Zuordnung zu der oder den jeweiligen Klasse oder Klassen erfolgen. Dies kann sowohl in einem analogen als auch einem digitalen Stromkreis realisiert werden.

Gemäß einer bevorzugten Ausgestaltung kann ein Speicher vorgesehen sein, und das Elektronikbauteil kann ausgebildet sein, gemessenen Zugkraftwerte, insbesondere regelmäßigen Intervallen gemessene Zugkraftwerte, fortlaufend in den Speicher abzulegen. Auf diese Weise kann die Vorrichtung eine im Nachhinein ablesbare bzw. abrufbare Aufzeichnung von Zugkräften erstellen. Alternativ oder zusätzlich kann der Speicher aufzeichnen, wie lange sich die Zugkraft innerhalb welchen Zugkraftbereiches befand, beispielsweise anhand der Zuordnung zu den jeweiligen Klassen.

Vorzugsweise ist ein Speicher vorgesehen, in dem Zugkräfte hinterlegt sind. Mittels des Speichers sind jeder Zugkraft jeweils eine Klasse der Mehrzahl von Klassen zugeordnet, wobei eine der Klassen mindestens eine der hinterlegten Zugkräfte, der oder denen sie zugeordnet ist, diese als für den Betrieb angemessen klassifiziert. In diesem Fall ist das Elektronikbauteil vorzugsweise ausgebildet, die gemessene Zugkraft mit den im Speicher hinterlegten Zugkräften zu vergleichen und auf diese Weise die ihr zugeordnete Klasse zu ermitteln. Bevorzugterweise sind bestimmte Zugkraftbereiche jeweils einer Klasse zugeordnet.

Bevorzugt sind die hinterlegten Zugkräfte in für den Betrieb angemessene Zugkräfte und in für den Betrieb unangemessene Zugkräfte klassifiziert, letztere insbesondere in für den Betrieb zu hohe Zugkräfte und in für den Betrieb zu niedrige Zugkräfte. Bevorzugt sind drei Klassen in der Vorrichtung insbesondere in dem Speicher hinterlegt, nämlich eine Klasse, die für den Betrieb angemessenen Zugkräften zugeordnet ist, eine weitere Klasse, die für den Betrieb zu hohen Zugkräften zugeordnet ist, und eine noch weitere Klasse, die für den Betrieb zu niedrigen Zugkräften zugeordnet ist.

Damit kann dem Benutzer effektiv und einfach angezeigt werden, ob er eine geeignete Wirkung mittels der für den Betrieb angemessenen Zugkräfte, eine schädigende Wirkung mittels der für den Betrieb zu hohen Zugkräfte oder eine unzufriedenstellende oder keine Wirkung mittels der für den Betrieb zu niedrigen Zugkräfte mittels Benutzung der Vorrichtung erzielt. Bei Penisextensionsgeräten wird die eingestellte Zugkraft üblicherweise in g (Gramm) angegeben. Beispielsweise kann als geeigneter Zugkraft-Bereich ein Bereich zwischen 600g bis 800g angesehen werden, während eine Zugkraft von weniger als 500g oder 600g als zu niedrig und eine Zugkraft von mehr als 800g oder 900g als zu hoch angesehen wird.

Vorzugsweise ist die Ausgabeeinrichtung als optische Anzeigeeinrichtung zur Wiedergabe von Farbinformationen ausgebildet. Die Anzeigeeinrichtung ist bevorzugt als elektronische Anzeigemittel beispielsweise in Form eines Displays und/oder als Leuchtmittel, insbesondere als LED (Leuchtdiode)-Leuchtmittel, ausgebildet. In einer bevorzugten Ausführungsform ist die Ausgabeeinrichtung als Balkenanzeige und/oder als mehrfarbige Leuchtdiode ausgebildet. Dadurch kann sie mehrere Klassen anzeigen. Bevorzugter ist die Anzeigeeinrichtung als mehrfarbig ansteuerbares Leuchtmittel, insbesondere als Leuchtdioden-Einrichtungen ausgebildet. Bevorzugter ist die Anzeigeeinrichtung als RGB-LED-Einrichtung ausgebildet, die je nach Ansteuerung eine große Zahl unterschiedlicher Farben und/oder Farbverläufe darstellen kann. Dadurch ist die Anzeigeeinrichtung zugleich kostengünstig, funktional flexibel bei einem geringen Stromverbrauch ausgestaltet.

Bevorzugt weist die Vorrichtung weiterhin ein Eingabeelement, insbesondere eine Eingabetaste auf, das ausgebildet ist, die Ausgabeeinrichtung zu aktivieren und/oder zu erlauben, die Zuordnung einer Klasse zu den hinterlegten Zugkräften einzustellen. Dadurch, dass der Benutzer die Aktivierung der Ausgabeeinrichtung steuert, ist die Vorrichtung stromsparend ausgebildet. Alternativ oder zusätzlich kann der Benutzer die Zuordnung einer Klasse zu den hinterlegten Zugkräften einstellen. Dadurch kann der Benutzer die Zuordnung der jeweiligen Klasse zu einer der hinterlegten Zugkräften in Abhängigkeit der Konstitution seines Penis hinterlegen und/oder ändern. Bevorzugter ist das Eingabeelement ausgebildet, zu erlauben, die zu der jeweilig hinterlegten Zugkraft zugeordnete Klasse zu ändern. D.h., die Klassen sind hinterlegt, können aber von dem Benutzer bei Bedarf geändert werden.

In einer bevorzugten Ausführungsform ist das Eingabeelement ausgebildet, in Abhängigkeit von einer Zeitdauer, in der das Eingabeelement betätigt wird, die Ausgabeeinrichtung zu aktivieren oder zu erlauben, die Zuordnung der Klasse zu den hinterlegten Zugkräften einzustellen. Dadurch sind beide Funktionen auf einfache Weise realisiert. Z.B. ist das Eingabeelement ausgebildet, die Ausgabeeinrichtung zu aktivieren, wenn es über eine vorbestimmte Zeitdauer betätigt wird, und zu erlauben, die Zuordnung der Klasse zu den hinterlegten Zugkräften einzustellen, wenn sie über eine weitere vorbestimmte Zeitdauer gedrückt wird, die von der vorbestimmten Zeitdauer verschieden ist. Z.B. ist die vorbestimmte Zeitdauer 1 Sekunde, während die weitere vorbestimmte Zeitdauer 3 Sekunden beträgt.

Bei dem Eingabeelement kann es sich in der einfachsten Form um eine Taste oder einen Schalter handeln. Auch kann es sich bei dem Eingabeelement um eine Tastatur handeln. Vorteilhaft sind auch andere geeignete Eingabeelemente wie Berührungssensoren, Näherungssensoren oder Touchpads. Insbesondere kann können die Ausgabeeinrichtung und das Eingabeelement zu einem Touch-Display kombiniert sein.

In einer bevorzugten Ausführungsform sind die Zugkraftmessvorrichtung, der Speicher, das Elektronikbauteil, die Stromquelle und die Ausgabeeinrichtung mit dem Befestigungselement und/oder der Zugvorrichtung fest verbunden. Bevorzugter sind sie mit der Zugvorrichtung fest verbunden. Dadurch bilden alle genannten Bauteile der Vorrichtung eine feststehende Einheit. Dadurch wird sichergestellt, dass die Zugkraftmessvorrichtung, der Speicher, das Elektronikbauteil, die Stromquelle und die Ausgabeeinrichtung fest in die Penisverlängerungs- und/oder -begradigungseinrichtung integriert ist.

In einer bevorzugten Ausführungsform ist der Speicher weiterhin ausgebildet, eine Betriebsdauer und/oder eine Anwendungsdauer der Vorrichtung des Elektronikbauteils zu speichern, und/oder die Ausgabeeinrichtung ausgebildet ist, die Betriebsdauer und/oder die Anwendungsdauer der Vorrichtung auszugeben. Dadurch kann dem Benutzer sein Benutzerverhalten insbesondere seine Benutzungsdauer kommuniziert werden.

Bevorzugt ist das Elektronikbauteil ausgebildet, die Ausgabeeinrichtung derart zu regeln oder zu steuern, dass sie die Betriebsdauer und/oder die Anwendungsdauer der Vorrichtung ausschließlich nach Eingabe eines vorbestimmten Codes ausgibt. Dadurch wird sichergestellt, dass nur eine autorisierte Person in diese Daten einsehen kann.

Die Ausgabeeinrichtung ist ausgebildet, die Benutzungsdauer visuell und/oder akustisch auszugeben. Bevorzugt ist die Ausgabeeinrichtung ausgebildet, die Betriebsdauer und/oder die Anwendungsdauer der Vorrichtung in Form einer optischen Codierung auszugeben, die ein Blinkmuster aufweist. Blinkmuster sind für einen Benutzer leicht unterscheid- und einprägsam. Bevorzugt wird das Blinkmuster durch Aktivieren von ein- oder verschiedenfarbigen LEDs der Ausgabeeinrichtung zu unterschiedlichen Zeitpunkten erzeugt. Z.B. ist die Ausgabeeinrichtung ausgebildet, mit jedem Blinken ein vorbestimmtes Zeitintervall anzuzeigen. Z.B. zeigt jedes Blinken eine Benutzungsdauer von einer Stunde an, so dass achtmaliges Blinken eine Benutzungsdauer von acht Stunden am Tag anzeigt. Alternativ kann die Ausgabeeinrichtung ebenfalls ausgebildet sein, die Benutzungsdauer akustisch über Sprachinformationen oder eine Toncodierung z.B. über eine Reihe aufeinanderfolgende Töne auszugeben.

Im vorliegenden Sinne beschriebt die Betriebsdauer den Zeitraum, über dem die Vorrichtung eingeschaltet ist, während die Anwendungsdauer vorzugsweise den Zeitraum beschreibt, während der eine Zugkraft auf den Penis einwirkt, vorzugsweise eine durch das Elektronikbauteil als angemessen klassifizierte Zugkraft. Bevorzugterweise wird in dem Speicher eine oder mehrere der folgenden Informationen gespeichert und/oder die Ausgabeeinrichtung ist ausgebildet, eine oder mehrere der folgenden Informationen auszugeben: Die Betriebsdauer, die Anwendungsdauer, den Zeitraum währenddessen die gemessene Zugkraft als einer ersten, zweiten und/oder einer dritten Klasse zugeordnet ermittelt wird, wobei die erste Klasse einer angemessenen Klassifizierung entspricht, während die zweite und die dritte Klasse jeweils einer zu geringen und einer zu hohen Zugkraft zugeordnet entsprechen.

Dass die Vorrichtung sich in der Anwendung und nicht lediglich im Betrieb befindet, kann beispielsweise mittels eines Schwellwertvergleichs ermittelt werden. Hierbei kann z.B. die Vorrichtung als in Anwendung angesehen werden, wenn die Zugkraft einen bestimmten Minimalwert, beispielsweise 50g, übersteigt.

Das Befestigungselement und die Vorrichtung sind bevorzugt derart ausgebildet, dass sie an unterschiedliche Penislängen anpassbar sind. Sie bilden zusammen eine Penisverlängerungs- und/oder -begradigungseinrichtung aus, d. h. eine Einheit, die eine Penisverlängerungs- und/oder -begradigungsfunktion bereitstellt. Penisverlängerungs- und/oder -begradigungseinrichtungen können auf verschiedene Weise implementiert sein und sind aus dem Stand der Technik bekannt. Bei Betrieb stellt der Benutzer die Zugkraft, die auf zumindest einen Teil des Penis ausgeübt wird, mittels der Zugkraftvorrichtung selbst ein.

Der Penis weist verschiedene Abschnitte auf. Er weist insbesondere einen Proximalabschnitt, einen Zwischenabschnitt und einen Distalabschnitt auf. Der Proximalabschnitt ist ein Abschnitt des Penis, der am nächsten zum Körper, insbesondere vom Körper ausgehend, angeordnet ist und weist bevorzugt den Penisansatz auf. Der Zwischenabschnitt ist bevorzugt ein Abschnitt, der zwischen dem Proximalabschnitt und dem Distalabschnitt liegt, wie der Penisschaft. Der Distalabschnitt ist ein Abschnitt des Penis, der gegenüber dem Proximalabschnitt und dem Zwischenabschnitt vom Körper entfernt angeordnet ist. Er umfasst beispielsweise die Glans Penis und/oder einen dazu angrenzenden Bereich.

Das Befestigungselement ist ausgebildet ist, den Penis des Benutzers zu befestigen. Das Befestigungselement ist bevorzugt mehrteilig ausgebildet. Bevorzugt weist es ein Stützelement, bevorzugter einen Stützring, zum Anordnen an einem Proximalabschnitt des Penis und ein Fixiermittel auf, das ausgebildet ist, einen Distalabschnitt des Penis zu fixieren. Dadurch wird eine sichere Befestigung des Penis an der Vorrichtung gewährleistet. Das Stützelement dient dazu, die Vorrichtung am Körper des Nutzers abzustützen.

Die Zugvorrichtung, die mit dem Befestigungselement verbunden ist und ausgebildet ist, eine Zugkraft auf zumindest einen Teil des Penis auszuüben, ist bevorzugt in Form von sich parallel erstreckenden Streckstangen ausgebildet. Die Streckstangen erstrecken sich bevorzugt parallel zum Zwischenabschnitt, weg von dem Stützelement. Die Streckstangen können gelenkig oder starr ausgebildet sein. Ferner können sie jeweils ein- oder mehrteilig ausgebildet sein.

Die Zugkraftmessvorrichtung, die ausgebildet ist, die bei Betrieb auf den zumindest einen Teil des Penis ausgeübte Zugkraft zu messen, kann auf verschiedene Weise implementiert sein. Zugkraftmessvorrichtungen sind allgemein bekannt.

Der Speicher, in dem Zugkräfte hinterlegt sind, denen jeweils eine Klasse einer Mehrzahl von Klassen zugeordnet sind, ist bevorzugt als ein interner Speicher der Vorrichtung ausgebildet. D. h., er ist nicht transportabel, sondern ist als ein fester Speicher der Vorrichtung ausgebildet. Der Speicher kann als Teil des Elektronikbauteils oder ein dazu separates Bauteil ausgebildet sein.

Das Elektronikbauteil, das ausgebildet ist, die gemessene Zugkraft mit den im Speicher hinterlegten Zugkräften zu vergleichen und die ihnen zugeordnete Klasse zu ermitteln, kann z. B. als ein Mikrocontroller ausgebildet sein. Das Elektronikbauteil enthält eine Regel- und/oder Steuereinheit, welche ausgebildet ist, die Zugkraftmessung, den Vergleich der gemessenen Zugkraft mit den im Speicher hinterlegten Zugkräften und die Ermittlung der zugeordneten Klasse und die Ausgabe der ermittelten Klasse mittels der Ausgabeeinrichtung zu regeln oder zu steuern.

Die Stromquelle, die zum Betreiben des Elektronikbauteils und optional des Speichers ausgebildet ist, ist eine Batterie oder bevorzugt ein wiederaufladbarer Energiespeicher.

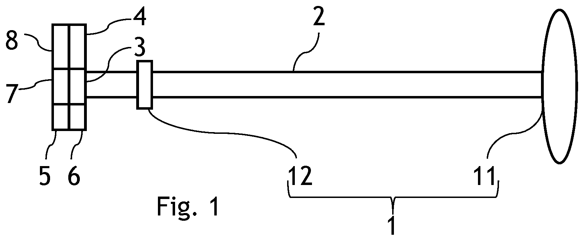

Nachfolgend wird die Erfindung unter Bezugnahme auf eine beigefügte Zeichnung detaillierter erläutert. Es zeigt schematisch und nicht maßstabsgerecht
- Fig.1: eine Seitenansicht einer erfindungsgemäßen Vorrichtung.

Fig.1 zeigt eine Seitenansicht einer erfindungsgemäßen Vorrichtung. Die Vorrichtung ist zur Verlängerung und/oder Begradigung eines Penis bestimmt und enthält ein Befestigungselement 1, das ausgebildet ist, einen Penis zu befestigen. Das Befestigungselement 1 weist ein Stützelement 11 in Form eines Stützrings zum Anordnen an einem Proximalabschnitt (nicht gezeigt) eines Penis (nicht gezeigt) und ein Fixiermittel 12 auf, das ausgebildet ist, einen Distalabschnitt (nicht gezeigt) des Penis zu fixieren. Ferner weist die Vorrichtung eine Zugvorrichtung 2 in Form sich parallel erstreckender Streckstangen auf. Die Streckstangen können ein- oder mehrteilig sein und geradlinig verlaufen oder gelenkig sein, hier sind sie beispielhaft geradlinig verlaufend gezeigt. Die Zugvorrichtung 2 ist mit dem Befestigungselement 1 verbunden und ausgebildet, eine Zugkraft auf zumindest einen Teil des Penis auszuüben. Das Befestigungselement 1 und die Zugvorrichtung 2 dienen als Penisverlängerungs- und/oder -begradigungseinrichtung.

Die Vorrichtung weist weiterhin eine Zugkraftmessvorrichtung 3 auf, die ausgebildet ist, die bei Betrieb auf den zumindest einen Teil des Penis ausgeübte Zugkraft zu messen, und einen Speicher 4 auf, in dem Zugkräfte hinterlegt sind, denen jeweils eine Klasse einer Mehrzahl von Klassen zugeordnet sind. Eine der Klassen klassifiziert mindestens eine der hinterlegten Zugkräfte, der oder denen sie zugeordnet ist, als für den Betrieb angemessen. Weiterhin weist die Vorrichtung ein Elektronikbauteil 5, das ausgebildet ist, die gemessene Zugkraft mit den im Speicher hinterlegten Zugkräften zu vergleichen und die ihnen zugeordnete Klasse zu ermitteln, eine Stromquelle 6, zum Betreiben des Elektronikbauteils 5, und eine Ausgabeeinrichtung 7 auf, die ausgebildet ist, die von dem Elektronikbauteil ermittelte Klasse akustisch und/oder visuell auszugeben, wenn die Zugkraft als für den Betrieb angemessen klassifiziert. Das Elektronikbauteil 5 weist eine Regel- und/oder Steuereinrichtung (nicht gezeigt) auf, die ausgebildet ist, die Zugkraftmessung, den Vergleich der gemessenen Zugkraft mit den im Speicher 4 hinterlegten Zugkräften und die Ermittlung der zugeordneten Klasse und die Ausgabe der ermittelten Klasse mittels der Ausgabeeinrichtung 7 zu regeln zu steuern.

Die Zugkraftmessvorrichtung 3, der Speicher 4, das Elektronikbauteil 5, die Stromquelle 6 und die Ausgabeeinrichtung 7 bilden eine Einrichtung, die mit der Zugvorrichtung 2 fest verbunden sind.

### Bezugszeichenliste:

- 1: Befestigungselement
- 11: Stützelement
- 12: Fixierelement
- 2: Zugvorrichtung
- 3: Zugmessvorrichtung
- 4: Speicher
- 5: Elektronikbauteil
- 6: Stromquelle
- 7: Ausgabeeinrichtung
- 8: Eingabeelement

## Patentansprüche

1. Vorrichtung zur Verlängerung und/oder Begradigung eines Penis, enthaltend:
- ein Befestigungselement (1), das ausgebildet ist, einen Penis zu befestigen,
- eine Zugvorrichtung (2), die mit dem Befestigungselement (1) verbunden ist und ausgebildet ist, eine Zugkraft auf zumindest einen Teil des Penis auszuüben,
- eine Zugkraftmessvorrichtung (3), die ausgebildet ist, die bei Betrieb auf den zumindest einen Teil des Penis ausgeübte Zugkraft zu messen,
- ein Elektronikbauteil (5), das ausgebildet ist, eine der gemessenen Zugkraft zugeordnete Klasse aus einer Mehrzahl an Klassen zu ermitteln, wobei die mehreren Klassen eine Klasse umfassen, welche eine ihr zugeordnete Zugkraft als für den Betrieb angemessen klassifiziert,
- eine mit dem Befestigungselement (1) und/oder der Zugvorrichtung (2) verbundene Ausgabeeinrichtung (7), die ausgebildet ist, die von dem Elektronikbauteil (5) ermittelte Klasse haptisch, akustisch und/oder visuell auszugeben, wenn sie die gemessene Zugkraft als für den Betrieb angemessen klassifiziert, und
- eine Stromquelle (6) zum Betreiben des Elektronikbauteils (5), der Zugkraftmessvorrichtung (3) und der Ausgabeeinrichtung (7).

2. Vorrichtung nach Anspruch 1, **gekennzeichnet durch** einen Speicher (4), in dem Zugkräfte hinterlegt sind, denen jeweils eine Klasse der Mehrzahl von Klassen zugeordnet sind, wobei eine der Klassen mindestens eine der hinterlegten Zugkräfte, der oder denen sie zugeordnet ist, diese als für den Betrieb angemessen klassifiziert, wobei das Elektronikbauteil (5) ausgebildet ist, die gemessene Zugkraft mit den im Speicher hinterlegten Zugkräften zu vergleichen und die ihr zugeordnete Klasse zu ermitteln.

3. Vorrichtung nach Anspruch 1 oder 2, **dadurch gekennzeichnet, dass** die Ausgabeeinrichtung (7) weiterhin ausgebildet ist, die von dem Elektronikbauteil (5) ermittelte Klasse haptisch, akustisch und/oder visuell auszugeben, wenn sie die gemessene Zugkraft als für den Betrieb unangemessen klassifiziert, wobei die Ausgabe der ermittelten Klasse, die die gemessene Zugkraft als für den Betrieb unangemessen klassifiziert, sich haptisch, akustisch und/oder visuell von der Ausgabe der Klasse, die die gemessene Zugkraft als für den Betrieb angemessen klassifiziert, unterscheidet.

4. Vorrichtung nach einem der vorangehenden Ansprüche, **dadurch gekennzeichnet, dass** die Ausgabeeinrichtung (7) als eine optische Anzeigeeinrichtung ausgebildet ist, die ausgebildet ist, die ermittelte Klasse mittels einer optischen Codierung auszugeben, die Farb- und/oder Symbolinformationen aufweist.

5. Vorrichtung nach Anspruch 4, **dadurch gekennzeichnet, dass** die Ausgabeeinrichtung (7) als Balkenanzeige und/oder als mehrfarbige Leuchtdiode ausgebildet ist.

6. Vorrichtung nach einem der vorangehenden Ansprüche, soweit auf Anspruch 2 rückbezogen, **gekennzeichnet durch** ein Eingabeelement (8), insbesondere eine Eingabetaste (8), das ausgebildet ist, die Ausgabeeinrichtung (7) zu aktivieren und/oder zu erlauben, die Zuordnung einer Klasse zu den hinterlegten Zugkräften einzustellen.

7. Vorrichtung nach Anspruch 6, **dadurch gekennzeichnet**, soweit auf Anspruch 2 rückbezogen, dass das Eingabeelement (8) ausgebildet ist, in Abhängigkeit von einer Zeitdauer, in der es betätigt wird, die Ausgabeeinrichtung (7) zu aktivieren und/oder zu erlauben, die Zuordnung der Klasse zu den hinterlegten Zugkräften einzustellen.

8. Vorrichtung nach einem der vorangehenden Ansprüche, soweit auf Anspruch 2 rückbezogen, **dadurch gekennzeichnet, dass** der Speicher (4) weiterhin ausgebildet ist, eine Betriebsdauer des Elektronikbauteils (5) und/oder eine Anwendungsdauer der Vorrichtung zu speichern, und die Ausgabeeinrichtung (7) ausgebildet ist, die Betriebsdauer auszugeben.

9. Vorrichtung nach Anspruch 8, **dadurch gekennzeichnet, dass** das Elektronikbauteil (5) ausgebildet ist, die Ausgabeeinrichtung (7) derart zu regeln oder zu steuern, dass sie die Betriebsdauer und/oder eine Anwendungsdauer der Vorrichtung ausschließlich nach Eingabe eines vorbestimmten Codes ausgibt.

10. Vorrichtung nach Anspruch 8 oder 9, **dadurch gekennzeichnet, dass** die Ausgabeeinrichtung (7) ausgebildet ist, die Betriebsdauer und/oder eine Anwendungsdauer der Vorrichtung in Form einer optischen Codierung auszugeben, die ein Blinkmuster aufweist.
